(19) Europäisches Patentamt
European Patent Office
Office européen des brevets

(11) Veröffentlichungsnummer: **0 178 503 A1**

# (12) EUROPÄISCHE PATENTANMELDUNG

(21) Anmeldenummer: 85112140.0

(22) Anmeldetag: 25.09.85

(51) Int. Cl.[4]: C 07 D 295/08
C 07 D 275/02, A 61 K 31/395
A 61 K 31/425

(30) Priorität: 17.10.84 DE 3438073

(43) Veröffentlichungstag der Anmeldung: 23.04.86 Patentblatt 86/17

(84) Benannte Vertragsstaaten: AT BE CH DE FR GB IT LI LU NL SE

(71) Anmelder: LUDWIG HEUMANN & CO GMBH
Heideloffstrasse 18-28 Postfach 2260
D-8500 Nürnberg(DE)

(72) Erfinder: Mörsdorf, Peter, Dr. Dipl.-Chem.
Untere Bahnhofstrasse 16
D-8501 Cadolzburg(DE)

(72) Erfinder: Schickaneder, Helmut, Dr. Dipl.-Chem.
Moosäcker 25
D-8501 Eckental(DE)

(72) Erfinder: Herter, Rolf, Dr. Dipl.-Chem.
Bayernstrasse 42
D-8540 Schwabach(DE)

(72) Erfinder: Postius, Stefan, Dr.
Nunnenbeckstrasse 24
D-8500 Nürnberg(DE)

(72) Erfinder: Szelenyi, Istvan, Dr.
Haendelstrasse 32
D-8501 Schwaig(DE)

(72) Erfinder: Ahrens, Kurt Henning, Dr.
Praterstrasse 9
D-8500 Nürnberg(DE)

(74) Vertreter: Kraus, Walter, Dr. et al,
Patentanwälte Kraus, Weisert & Partner
Thomas-Wimmer-Ring 15
D-8000 München 22(DE)

**(54) Butenylderivate, Verfahren zu ihrer Herstellung und diese Verbindungen enthaltende Arzneimittel.**

(57) Butenylderivate, welche eine hohe selektive Wirkung auf Histamin-$H_2$-Rezeptoren haben der Formel

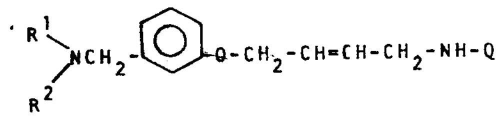

in der $R^1$ und $R^2$, die gleich oder verschieden sind, für Wasserstoff, lineares oder verzweigtes Alkyl oder Cycloalkyl stehen oder $R^1$ und $R^2$ zusammen mit dem Stickstoffatom, an das sie gebunden sind, einen stickstoffhaltigen alicyclischen Heterocyclus bilden, Q für die Gruppierung

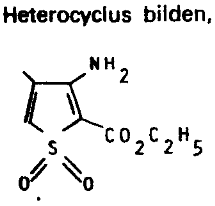

oder

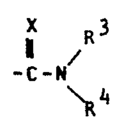

steht, worin X ein Sauerstoffatom oder die Gruppe N-CN oder $CH-NO_2$ bedeutet. $R^3$ und $R^4$ unabhängig voneinander für Wasserstoffatom, unsubstituierte oder durch eine Hydroxylgruppe substituierte lineare oder verzweigte Alkylgruppe oder eine Propargylgruppe stehen oder $R^3$ und $R^4$ zusammen mit dem Stickstoffatom, an das sie gebunden sind, einen alicyclischen stickstoffhaltigen Heterocyclus bilden, und Verfahren zu deren Herstellung.

EP 0 178 503 A1

Croydon Printing Company Ltd.

Die Erfindung betrifft neue Butenylderivate mit einer hohen selektiven Wirkung auf Histamin-$H_2$-Rezeptoren, Verfahren zu ihrer Herstellung und Arzneimittel, die diese Verbindungen enthalten, sowie schließlich die Verwendung dieser Verbindungen in der Therapie.
Als Anti-Ulcusmittel haben Cimetidin und Ranitidin bereits Eingang in die Therapie gefunden. Die Halbwertszeiten von Cimetidin und Ranitidin sind jedoch verhältnismäßig kurz. Dadurch wird es nötig, mehrfach täglich Tabletten mit Dosiseinheiten von 160 - 300 mg in einer therapeutisch festgelegten Form zu verabreichen. Es besteht weiterhin ein Bedarf an Anti-Ulcusmitteln, die in ihrem pharmakologischen Wirkprofil dem Cimetidin und Ranitidin überlegen sind. Die erfindungsgemäßen Verbindungen zeigen aufgrund ihrer spezifischen $H_2$-antagonistischen Aktivität eine Hemmung der Magensäuresekretion, wenn diese durch Histaminagonisten stimuliert wird. Ash und Shild, "Brit. J. Pharmacol. Chemother.", 27, 427 (1966) und Black et al., "Nature", 236, 385 (1972).
Die pharmakologische Aktivität dieser Verbindungen kann nach einer modifizierten Methode gemäß der DE OS 2734070 beim perfundierten Rattenmagen gezeigt werden oder durch die Ermittlung der $pA_2$-Werte in vitro am Meerschweinchenvorhof (vergleiche Ariens, Molec. Pharmac., Band 1, Academic Press, New York, 1964) nachgewiesen werden.
Weiterhin kann die $H_2$-antagonistische Wirkung an wachen Heidenhain-Pouch-Hunden nach der Methode von Black et al., "Nature" 236, 385 (1972) und an wachen Fistelkatzen gezeigt werden.
Die neuen Verbindungen antagonisieren weiterhin die Histaminwirkung auf die Kontraktionsfrequenz des isolierten rechten Atriums des Meerschweinchens, aber sie beeinflussen nicht histamininduzierte Kontraktionen des isolierten, glatten gastrointestinalen Muskels, wenn diese durch $H_2$-Agonisten hervorgerufen werden. Nachdem Hemmstoffe für Histamin-$H_2$-Rezeptoren sowohl bezüglich der basalen Magensäuresekretion als auch der durch Gastrin, Histamin, Metacholin oder Nahrung induzierten Magensäuresekretion Hemmwirkung besitzen, können sie für die Behandlung von peptischen Ulcera, die durch übermäßige Sekretion von Magensäure verursacht sind,

und bei der Therapie hyperacider Gastritis verwendet werden. Der Erfindung liegt die Aufgabe zugrunde, neue Hemmstoffe für Histamin-$H_2$-Rezeptoren mit verbesserter Wirksamkeit zur Verfügung zu stellen. Diese Aufgabe wird durch die Erfindung gelöst.

Gegenstand der Erfindung sind daher neue Butenylderivate der allgemeinen Formel I

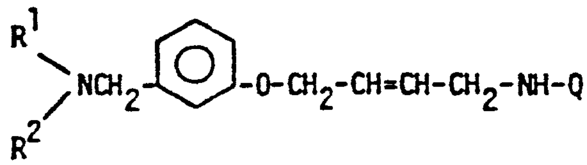 (I)

in der $R^1$ und $R^2$, die gleich oder verschieden sein können, jeweils für Wasserstoff, lineares oder verzweigtes $C_1$-$C_6$-Alkyl oder $C_5$-$C_6$-Cycloalkyl stehen oder $R^1$ und $R^2$ zusammen mit dem Stickstoffatom, an das sie gebunden sind, einen 4- bis 7gliedrigen stickstoffhaltigen alicyclischen Heterocyclus bilden, Q für die Gruppierung

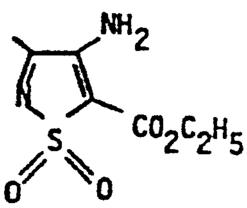 oder 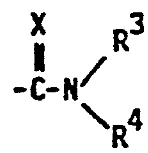

steht, worin X ein Sauerstoffatom oder die Gruppe N-CN oder CH-$NO_2$ bedeutet, $R^3$ und $R^4$ unabhängig voneinander jeweils für ein Wasserstoffatom, eine unsubstituierte oder durch eine Hydroxylgruppe substituierte lineare oder verzweigte $C_1$-$C_6$-Alkylgruppe oder eine Propargylgruppe stehen oder $R^3$ und $R^4$ zusammen mit dem Stickstoffatom, an das sie gebunden sind, einen 4- bis 6gliedrigen alicyclischen stickstoffhaltigen Heterocyclus bilden, sowie ihre physiologisch annehmbaren Hydrate und Salze.

In der allgemeinen Formel I bedeuten $R^1$ und $R^2$, die gleich oder verschieden sein können, jeweils ein Wasserstoffatom, eine lineare oder verzweigtkettige Alkylgruppe mit 1 bis 6 Kohlenstoffatomen, vorzugsweise 1 bis 3 Kohlenstoffatomen, oder eine $C_5$-$C_6$-Cycloalkylgruppe. Beispiele für lineare oder verzweigtkettige Alkylgruppen mit 1 bis 6 Kohlenstoffatomen sind die Methyl-, Ethyl-, i- und n-Propyl-, i- und n-Butyl-, n-Pentyl- und n-Hexylgruppe. Bevorzugt werden die Methyl-, Ethyl-, i- und n-Propylgruppe. Besonders bevorzugt werden die Methyl- und Ethylgruppe. $R^1$ und $R^2$ können auch zusammen mit dem Stickstoffatom, an das sie gebunden sind, einen 4- bis 7gliedrigen, vorzugsweise 5- oder 6gliedrigen stickstoffhaltigen heterocyclischen Ring bilden. Beispiele für einen solchen Ring sind der Azetidin-, Pyrrolidin- und Piperidinring sowie der Homopiperidinring. Bevorzugt werden der Pyrrolidin- und Piperidinring.

Q bedeutet die Gruppierung

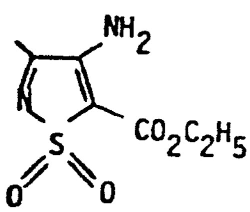

oder

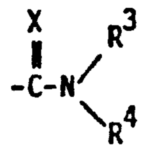

Im Falle der letztgenannten Gruppierung bedeutet X ein Sauerstoffatom oder die Gruppe $CH-NO_2$ oder N-CN, wobei die Gruppe $CH-NO_2$ bevorzugt wird.

Die Gruppen $R^3$ und $R^4$ stehen jeweils unabhängig voneinander für ein Wasserstoffatom, eine unsubstituierte oder durch eine Hydroxylgruppe substituierte lineare oder verzweigte $C_1$-$C_6$-Alkylgruppe, vorzugsweise $C_1$-$C_3$-Alkylgruppe, oder eine Propargylgruppe. Beispiele für die so definierten Alkylgruppen sind die oben definierten Gruppen, wobei auch in diesem Falle die Methyl- und Ethylgruppe ganz besonders bevorzugt werden. Die genannte $C_1$-$C_6$-Alkylgruppe, vorzugsweise $C_1$-$C_3$-Alkylgruppe, kann unsubstituiert sein oder durch eine Hydroxylgruppe, vorzugsweise in 2-Stellung, substituiert sein. $R^3$ und $R^4$ können ebenfalls mit dem Stickstoffatom, an das sie gebunden sind, einen 4- bis 6gliedrigen stickstoffhaltigen Ring, d.h. einen Azetidin-, Pyrrolidin- oder Piperidinring, bilden.

Eine bevorzugte Gruppe von erfindungsgemäßen Verbindungen ist dadurch gekennzeichnet, daß $R^1$, $R^2$ und Q die oben angegebenen Bedeutungen haben, daß X für $CH-NO_2$ steht, daß $R^3$ ein Wasserstoffatom bedeutet und daß $R^4$ eine lineare oder verzweigte $C_1$-$C_6$-Alkylgruppe, bevorzugt eine $C_1$-$C_3$-Alkylgruppe, wie zum Beispiel eine Methyl-, Ethyl-, Propyl- oder Isopropylgruppe, ganz besonders bevorzugt eine Methylgruppe, bedeutet.

Eine weitere bevorzugte Gruppe von erfindungsgemäßen Verbindungen ist dadurch gekennzeichnet, daß $R^1$, $R^2$ und Q die oben angegebenen Bedeutungen haben, daß X für die Gruppe N-CN steht, daß $R^3$ ein Wasserstoffatom ist und daß $R^4$ eine lineare $C_1$-$C_3$-Alkylgruppe, die in 2-Stellung durch eine Hydroxylgruppe substituiert ist, darstellt.

Eine andere bevorzugte Gruppe von erfindungsgemäßen Verbindung ist dadurch gekennzeichnet, daß $R^1$, $R^2$ und Q die oben angegebenen Bedeutungen haben und daß X für ein Sauerstoffatom und $R^3$ und $R^4$ jeweils für ein Wasserstoffatom stehen.

Die erfindungsgemäßen Verbindungen der allgemeinen Formel I können in stereoisomeren cis- und trans-Formen vorliegen, die alle unter den Rahmen der vorliegenden Erfindung fallen. Die trans-Verbindungen werden im allgemeinen bevorzugt.

Die erfindungsgemäßen Verbindungen werden durch ein Verfahren hergestellt, das dadurch gekennzeichnet ist, daß man

a) zur Herstellung von Verbindungen, bei denen $R^1$ und $R^2$ wie oben definiert sind und Q für die Gruppierung

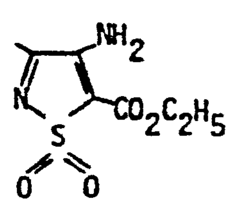

steht, ein Amin der allgemeinen Formel II

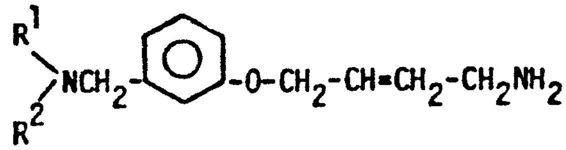

(II)

in der $R^1$ und $R^2$ wie oben definiert sind, mit einem Isothiazolderivat der Formel

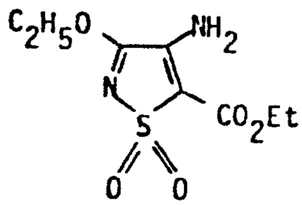

zu einer erfindungsgemäßen Verbindung der allgemeinen Formel I umsetzt, oder daß man

b) zur Herstellung von Verbindungen, bei denen $R^1$ und $R^2$ wie oben definiert sind und Q für die Gruppierung

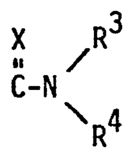

steht, worin X für N-CN oder $CH-NO_2$ steht, eine Verbindung der allgemeinen Formel III

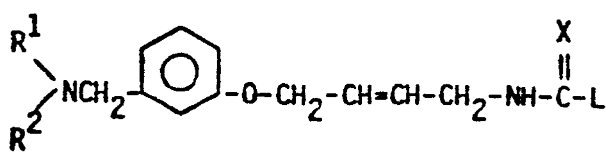

(III)

in der $R^1$ und $R^2$ wie oben definiert sind und L für $SCH_3$, $OCH_3$, $OC_2H_5$ oder O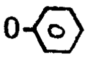 als Austrittsgruppe steht, mit einem Amin der allgemeinen Formel IV

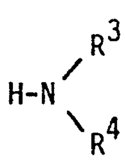

(IV)

in der $R^3$ und $R^4$ wie oben definiert sind, zu einer erfindungsgemäßen Verbindung der allgemeinen Formel I umsetzt, oder daß man

c) zur Herstellung von Verbindungen, bei denen $R^1$ und $R^2$ wie oben definiert sind und Q für die Gruppierung

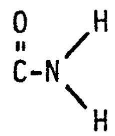

steht, eine Verbindung der allgemeinen Formel V

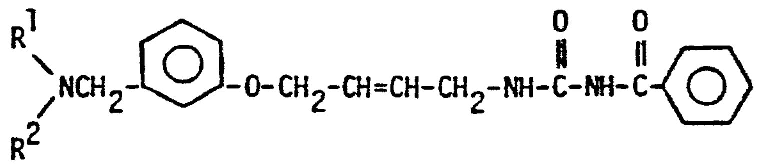

(V)

in der $R^1$ und $R^2$ die oben angegebenen Bedeutungen haben, zu einer erfindungsgemäßen Verbindung der allgemeinen Formel I umsetzt,

und daß man gegebenenfalls die gemäß a) bis c) erhaltene Verbindung in an sich bekannter Weise in ihr physiologisch annehmbares Salz umwandelt.

Das Verfahren gemäß der Variante a), wie oben definiert, wird vorzugsweise in einer alkoholischen Lösung, wie Methanol, Ethanol oder Isopropanol, durchgeführt. Die dabei erhaltenen Verbindungen der allgemeinen Formel I werden in an sich bekannter Weise aufgearbeitet und isoliert, beispielsweise durch Kristallisieren und Umkristallisieren.

Das Verfahren gemäß der Variante b) wird ebenfalls in einer alkoholischen Lösung, wie Methanol, Ethanol oder Isopropanol, durchgeführt. Auch die Aufarbeitung erfolgt wie im Zusammenhang mit der Variante a) beschrieben.

Die Variante c) wird vorzugsweise als basenkatalysierte Reaktion, insbesondere in einer wäßrigmethanolischen Kaliumcarbonatlösung, durchgeführt. Die Aufarbeitung und Isolierung erfolgt ebenfalls in an sich bekannter Weise. So können beispielsweise die isomeren Gemische durch fraktionierte Kristallisation getrennt werden.

Die Herstellung der Ausgangsprodukte erfolgt in an sich bekannter Weise.

Die Erfindung umfaßt auch die stereoisomeren Verbindungen der Formel I in Form von physiologisch annehmbaren Hydraten und Salzen mit anorganischen und organischen Säuren. Diese Salze können durch einfache Umsetzung mit Mineralsäuren, wie Chlor-, Brom- und Jodwasserstoffsäure, Phosphorsäure, Metaphosphorsäure, Salpetersäure oder Schwefelsäure, oder mit organischen Säuren, wie Ameisensäure, Essigsäure, Propionsäure, Phenylessigsäure, Weinsäure, Zitronensäure, Fumarsäure, Methansulfonsäure etc., in an sich bekannter Weise gebildet werden.

Die erfindungsgemäßen Verbindungen können zur Verabreichung in jeder beliebigen Weise formuliert werden. Die Erfindung umfaßt daher auch Arzneimittel, die mindestens eine erfindungsgemäße Verbindung zur Verwendung in der Human- oder Veterinärmedizin enthalten.

Solche Arzneimittel können herkömmlicherweise unter Verwendung von einem oder mehreren pharmazeutisch annehmbaren Trägern oder Verdünnungsmitteln hergestellt werden.

Die erfindungsgemäßen Verbindungen können daher für die orale, bukkale, topische, parenterale oder rektale Verabreichung formuliert werden, wobei die orale Verabreichung bevorzugt wird. Für die orale Verabreichung kann das Arzneimittel in Form von beispielsweise Tabletten, Kapseln, Pulvern, Lösungen, Sirups oder Suspensionen vorliegen, die unter Verwendung von annehmbaren Verdünnungsmitteln auf herkömmliche Weise hergestellt worden sind. Für die bukkale Verabreichung kann das Arzneimittel die Form von Tabletten oder Briefchen einnehmen, die in herkömmlicher Weise formuliert worden sind.

Die erfindungsgemäßen Verbindungen können für die parenterale Verabreichung durch Bolusinjektion oder kontinuierliche Infusion formuliert werden. Formulierungen zur Injektion können in Dosiseinheitsform als Ampullen oder in Mehrfachdosenbehältern mit zugesetztem Konservierungsmittel vorliegen.

Die Arzneimittel können solche Formen, wie Suspensionen, Lösungen oder Emulsionen in öligen oder wäßrigen Trägern, einnehmen, und sie können Formulierungshilfsmittel, wie Suspendierungs-, Stabilisierungs- und/oder Dispergierungsmittel, enthalten.

Alternativ kann der Wirkstoff auch in Pulverform zur Rekonstitution mit einem geeigneten Träger, zum Beispiel sterilem, pyrogenfreiem Wasser, vor dem Gebrauch vorliegen.

Die erfindungsgemäßen Verbindungen können auch für rektale Zubereitungen, zum Beispiel Suppositorien oder Retentionseinläufe, formuliert werden, die zum Beispiel herkömmliche Suppositoriengrundlagen, wie Kakaobutter oder andere Glyceride, enthalten.

Zur topischen Anwendung können die erfindungsgemäßen Verbindungen als Salben, Cremes, Gels, Lotionen, Pulver oder Sprays in herkömmlicher Weise formuliert werden.

Für die orale Verabreichung ist eine geeignete Tagesdosis an erfindungsgemäßen Verbindungen 1 bis 4 Dosen bis insgesamt 5 mg bis 1 g/Tag, vorzugsweise 5 bis 250 mg/Tag, je nach Zustand des Patienten. Im Einzelfall kann es gegebenenfalls erforderlich sein, von den genannten Mengen abzuweichen, und zwar in Abhängigkeit vom individuellen Verhalten gegenüber dem Wirkstoff bzw. der Art seiner Formulierung und dem Zeitpunkt bzw. Intervall, zu welchem die Verabreichung erfolgt. So gibt es zum Beispiel Fälle, wo mit weniger als der oben genannten Mindestmenge ausgekommen werden kann, während in anderen Fällen die genannte obere Grenze überschritten werden muß.

Die erfindungsgemäßen Verbindungen zeichnen sich gegenüber anerkannt guten Arzneimitteln der gleichen Wirkungsrichtung durch eine Verbesserung der pharmakologischen Aktivitäten aus. Dies ergibt sich aus den Ergebnissen der im folgenden dargestellten pharmakologischen Vergleichsuntersuchungen (erhalten nach der Methode von Ariens, Molec. Pharmac., Band 1, Academic Press, New York (1964)).

CIMETIDIN (Vergleich): $pA_2 = 6{,}2$
Beispiel 4 : $pA_2 = 7{,}5$

Die Erfindung wird anhand der folgenden Beispiele näher erläutert.

Beispiel 1

a) Herstellung von N-(4-Brombut-2-enyl)phthalimid

25 g (117 mmol) trans-1,4-Dibrom-2-buten und 18.1 g (98 mmol) Kaliumphthalimid in 150 ml Dimethylformamid werden 5 Std. bei 120 °C gerührt. Die Lösung wird zu 1 Ltr. Eiswasser gegeben und das Gemisch mit 3 x 200 ml Methylenchlorid extrahiert. Die organische Phase wird mit 250 ml 1 N Kalilauge, mit 200 ml ges. $NaHCO_3$-Lösung und 200 ml Wasser gewaschen und über Natriumsulfat getrocknet. Nach Abfiltrieren und Einengen i. Vak. wird der feste Rückstand in 300 ml heißem Ethanol aufgenommen. Nach Abkühlen auf Raumtemperatur wird der ausgefallene Niederschlag abgesaugt und die Mutterlauge i. Vak. eingeengt. Das verbleibende Rohprodukt wird aus Diisopropylether umkristallisiert.
8,0 g (29 %) farblose Nadeln vom Schmelzpunkt 97,0 - 98,5 °C.

| | | | | | |
|---|---|---|---|---|---|
| $C_{12}H_{10}BrNO_2$ (280.1) | Ber.: | C 51.45 | H 3.60 | N 5.00 | Br 28.53 |
| | Gef.: | C 51.57 | H 3.59 | N 4.94 | Br 28.44 |

$^1$H-NMR-Daten: ($CDCl_3$, TMS als interner Standard)

$\delta$ = 3.93 (d) 2 H,
4.32 (d) 2 H,
5.70 - 6.21 (m) 2 H,
7.71 - 8.02 (m) 4 H ppm.

b) Herstellung von N-[4-[3-(Piperidinomethyl)phenoxy]but-2-enyl]phthalimid

Zu einer Lösung von 8.0 g (28.6 mmol) N-(4-Brombut-2-enyl)phthalimid in 20 ml Dimethylformamid wird eine Lösung von 5.81g(28.6 mmol) Natrium-(3-piperidinomethyl)phenolat (hergestellt aus 5.47 g 3-(Piperi-dinomethyl)phenol und 0.85 g Natriumhydrid 80 %) in 45 ml Dimethylformamid getropft. Nach 3-stündigem Rühren bei 50 °C wird die Lösung auf 400 ml Eiswasser gegeben und die Mischung mit 3 x 100 ml Methylenchlorid extrahiert. Die organ. Phase wird mit Wasser gewaschen und über Natriumsulfat getrocknet. Nach Abfiltrieren des Trocknungsmittels und Einengen der Lösung i. Vak. verbleibt ein hellbraunes Öl, das mit Methylenchlorid/Ethanol 100:8 an Kieselgel chromatographiert wird. Die Hauptfraktion ergibt nach Einengen 7.45 g (67 %) der Titelverbindung als blaßgelbes Öl.

| | |
|---|---|
| $^1$H-NMR-Daten: | $\delta$ = 1.28 - 1.79 (m) 6 H, |
| ($CDCl_3$, TMS als | 2.31 - 2.56 (m) 4 H, |
| interner Standard) | 3.50 (s) 2 H, |
| | 4.30 - 4.45 (m) 2 H, |
| | 4.45 - 4.61 (m) 2 H, |
| | 5.95 - 6,09 (m) 2 H, |
| | 6.74 - 7.40 (m) 4 H, |
| | 7.71 - 8.03 (m) 4 H ppm. |

c) Herstellung von 1-Amino-4-[3-(piperidinomethyl)phenoxy]-2-buten

7.45 g (19.1 mmol) N-[4-[3-(Piperidinomethyl)phenoxy]but-2-enyl]phthalimid und 2.45 ml 80 % Hydrazinhydrat in 65 ml Ethanol werden 2 Std. unter Rückfluß gekocht. Nach Einengen im Vakuum wird der Rückstand in 50 ml Wasser aufgenommen und mit 7,5 ml konz. Salzsäure versetzt. Die nach Absaugen des Niederschlags verbleibende Mutterlauge wird mit Natriumhydroxid auf pH 13 eingestellt und mit 3 x 50 ml Methylenchlorid extrahiert. Die organische Phase ergibt nach Trocknen über Natriumsulfat und Einengen im Vakuum 4.97 g (100 %) eines blaßgelben Öls, das nach kurzer Zeit kristallisiert.

| | |
|---|---|
| $^1$H-NMR-Daten: | $\delta$ = 1.21 (breit) 2 H, austauschbar mit $D_2O$ |
| ($CDCl_3$, TMS als | 1.31 - 1.73 (m) 6 H, |
| interner Standard) | 2.21 - 2.50 (m) 4 H, |

3.30 - 3.45 (m) 2 H,
3.47 (s) 2 H,
4.45 - 4.67 (m) 2 H,
5.82 - 6.08 (m) 2 H,
6.70 - 7.37 (m) 4 H ppm.

d) Herstellung von $N^1$-Cyano-$N^2$-[4-[3-(piperidinomethyl)phenoxy]but-2-enyl]-O-phenyl-isoharnstoff

1.0 g (3.8 mmol) 1-Amino-4-[3-(piperidinomethyl)phenoxy]-2-buten und 0.9 g ( 3.8 mmol) N-Cyano-O,O-diphenyl-imidocarbonat, in 10 ml i-Propanol, werden 3 Std. bei 50 °C gerührt. Nach Abdampfen des Lösungsmittels im Vakuum wird der Rückstand in 50 ml Methylenchlorid aufgenommen und die Lösung 4 x mit 10 ml 1 N Kalilauge ausgeschüttelt. Die organische Phase wird mit 20 ml Wasser gewaschen, über Natriumsulfat getrocknet, filtriert und eingedampft. Man erhält 1.49 g (97 %) eines farblosen Öls, das langsam kristallisiert.

$^1$H-NMR-Daten: ($CDCl_3$, TMS als interner Standard)

$\delta$ = 1.35 - 1.70 (m) 6 H,
2.24 - 2.51 (m) 4 H,
3.46 (s) 2 H,
3.96 - 4.18 (m) 2 H,
4.46 - 4.63 (m) 2 H,
5.88 - 6.08 (m) 2 H,
6.72 - 7.70 (m) 9 H ppm.

e) Herstellung von $N^1$-Cyano-$N^2$-Methyl-$N^3$-[4-[3-(piperidinomethyl)phenoxy]but-2-enyl]guanidin

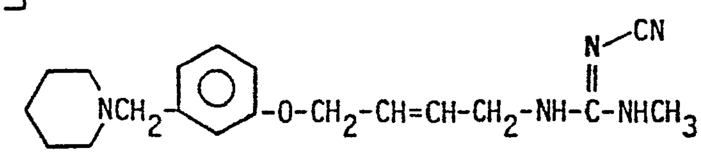

In eine Lösung von 1,5 g (3.7 mmol) $N^1$-Cyano-$N^2$-[4-[3-(piperidinomethyl)phenoxy]but-2-enyl]-O-phenyl-isoharnstoff in 20 ml Ethanol werden unter Eiskühlung 5 g Methylamin (ca. 160 mmol) eingeleitet. Nach Rühren über Nacht bei Raumtemperatur wird das Lösungsmittel im Vakuum abgedampft und der Rückstand in 30 ml Methylenchlorid aufgenommen. Die organische Phase wird dreimal mit 10 ml 1 N Kalilauge und einmal mit 20 ml Wasser gewaschen, über Natriumsulfat getrocknet, filtriert und im Vakuum eingedampft. Das verbleibende Öl wird mit Ethanol als Laufmittel an Kieselgel gereinigt. Die Hauptfraktion ergibt 750 mg (59 %) der Titelverbindung als blaßgelbes Öl.

$C_{19}H_{27}N_5O$ (341.5)

Rf (Ethanol): 0.58

$^1$H-NMR-Daten: (CDCl$_3$, TMS als interner Standard)

$\delta$ = 1.28 - 1.71 (m) 6 H,
2.23 - 2.51 (m) 4 H,
2.81 (d) 3 H, (-NH-C$\underline{H}_3$)
3.45 (s) 2 H, ( >N-C$\underline{H}_2$-Ar)
3.80 - 4.06 (m) 2 H,
4.42 - 4.63 (m) 2 H,
5.57 - 5.99 (m) 4 H, 2 H austauschbar mit $D_2O$
6.67 - 7.34 (m) 4 H ppm.

Beispiel 2

$N^1$-Cyano-$N^2$-[4-[3-(piperidinomethyl)phenoxy]but-2-enyl]guanidin

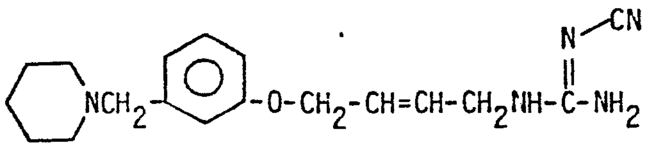

Zu einer Lösung von 1.55 g (3.8 mmol) $N^1$-Cyano-$N^2$-[4-[3-(piperidinomethyl)phenoxy]but-2-enyl]-O-phenyl-isoharnstoff in 10 ml Ethanol werden 20 ml ethanol. Ammoniak (5 Mol/l, 100 mmol) getropft . Nach 20 Std. bei Raumtemperatur wird die Lösung eingedampft, der Rückstand in 50 ml Methylenchlorid aufgenommen und mit 3 x 20 ml 10 %iger Natronlauge extrahiert. Nach Trocknen und Einrotieren der organischen Phase verbleibt ein blaßgelbes Öl, das nach Anreiben kristallisiert. Ausbeute 1.20 g (95 %), Schmelzpunkt 111 - 113 $^o$ C

$C_{18}H_{25}N_5O$ (327.4)

Rf (EtOH / $N(Et)_3$ 97 : 3): 0.62

| | |
|---|---|
| $^1$H-NMR-Daten: ($CDCl_3$, TMS als interner Standard) | $\delta$ = 1.23 - 1.71 (m) 6 H,<br>2.19 - 2.49 (m) 4 H,<br>3.40 (s) 2 H,<br>3.68 - 4.00 (m) 2 H,<br>4.34 - 4.58 (m) 2 H,<br>5.74 - 5.91 (m) 2 H,<br>6.01 (breit) 2 H, austauschbar mit $D_2O$<br>6.42 (breit) 1 H, austauschbar mit $D_2O$<br>6.67 - 7.32 (m) 4 H ppm. |

Beispiel 3

$N^1$-Cyano-$N^2$-(2-hydroxyethyl)-$N^3$-[4-[3-(piperidinomethyl)phenoxy]but-2-enyl]-guanidin

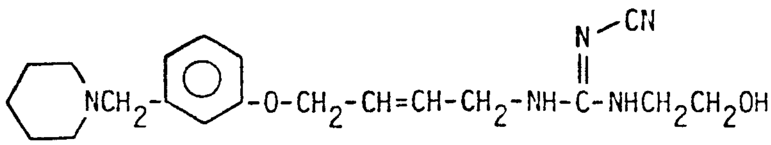

Zu einer Lösung von 1.55 g (3.8 mmol) $N^1$-Cyano-$N^2$-[4-[3-(piperidinomethyl)phenoxy]but-2-enyl]-O-phenyl-isoharnstoff in 10 ml Ethanol werden 0.70 g (11.5 mmol) 2-Aminoethanol gegeben. Die Mischung wird zunächst 16 Std. bei Raumtemperatur gerührt und anschließend 1 Std. auf 60 °C erwärmt. Nach Eindampfen der Lösung im Vakuum wird der Rückstand in 50 ml Methylenchlorid aufgenommen, mit 3 x 20 ml 10 %iger Natronlauge gewaschen und über Natriumsulfat getrocknet. Nach Eindampfen wird das erhaltene Öl mit Ethanol/Triethylamin 97 : 3 an Kieselgel chromatographiert. Man erhält nach Eindampfen der Hauptfraktion 1.25 g (88 %) der Titelverbindung als farbloses Öl.

$C_{20}H_{29}N_5O_2$ (371.5)

Rf (EtOH / $N(Et)_3$ 97 : 3): 0.70

$^1H$-NMR-Daten: (CDCl$_3$, TMS als interner Standard)

$\delta$ = 1.28 - 1.72 (m) 6 H,
2.23 - 2.52 (m) 4 H,
3.21 - 3.52 (m) 2 H,
3.43 (s) 2 H,
3.60 - 4.03 (m) 3 H, 1 H austauschbar mit $D_2O$
4.10 - 4.35 (m) 2 H,
4.42 - 4.60 (m) 2 H,
5.80 - 5.99 (m) 2 H,
6.30 (t, breit) 1 H, austauschbar mit $D_2O$
6.71 - 7.37 (m) 4 H ppm.

Beispiel 4

1-Methylamino-1-[4-[3-(piperidinomethyl)phenoxy]but-2-enyl]amino-2-nitro-ethylen

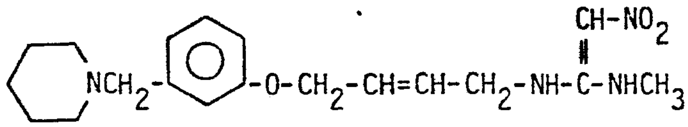

1.38 g (5.3 mmol) 1-Amino-4-[3-(piperidinomethyl)phenoxy]-2-buten und 0.88 g (5.3 mmol) 1,1-S,S-dimethyl-dithio-2-nitroethen werden in 15 ml Acetonitril 5 Std. unter Rückfluß gekocht. Nach Abdampfen des Lösungsmittels im Vakuum wird der Rückstand in 50 ml Methanol aufgenommen. In die unter Rückfluß siedende Lösung wird dann während 2 Std. ein kräftiger Strom trockenen Methylamins eingeleitet. Nach Eindampfen der Lösung wird das erhaltene Rohprodukt an Kieselgel mit Methanol/Essigester 80 : 20 chromatographiert. Man erhält 1.49 g (78 %) der Titelverbindung als blaßgelbes Öl.

$C_{19}H_{28}N_4O_3$ (360.4)

Rf (EtOH/N(Et)$_3$ 97 : 3): 0.52

$^1$H-NMR-Daten: (d$_6$-DMSO, TMS als interner Standard)

$\delta$ = 1.20 - 1.63 (m) 6 H,
2.12 - 2.41 (m) 4 H,
2.78 (s, breit) 3 H,
3.35 (s) 2 H,
3.76 - 4.01 (m) 2 H,
4.46 - 4.62 (m) 2 H,
5.79 - 5.96 (m) 2 H,
6.46 (s) 1 H,
6.70 - 7.30 (m) 4 H,
10.0 (breit) 2 H, austauschbar mit $D_2O$, ppm.

Beispiel 5

1-Amino-1-[4-[3-(piperidinomethyl)phenoxy]but-2-enyl]amino-2-nitroethylen

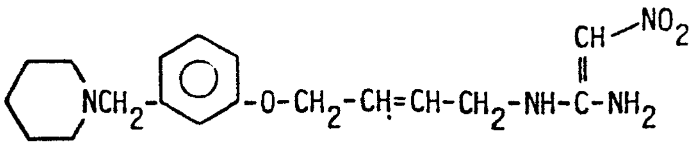

1.70 g (6.53 mmol) 1-Amino-4-[3-(piperidinomethyl)phenoxy]-2-buten und 1.08 g (6.53 mmol) 1,1-S,S-Dimethyl-dithio-2-nitroethen werden in 15 ml Acetonitril 5 Std. unter Rückfluß gekocht. Nach Abdampfen des Lösungsmittels im Vakuum wird der Rückstand in 50 ml Methanol aufgenommen. In die unter Rückfluß siedende Lösung wird dann während 2 Std. ein kräftiger Strom trockenen Ammoniaks eingeleitet. Nach Eindampfen der Lösung wird das erhaltene Rohprodukt an Kieselgel mit Methanol/Essigester 80 : 20 chromatographiert. Man erhält 1.90 g (84 %) der Titelverbindung als blaßgelbes Öl.

$C_{18}H_{26}N_4O_3$ (346.4)

Rf (MeOH): 0.46

| | |
|---|---|
| $^1$H-NMR-Daten: | $\delta$ = 1.27 - 1.77 (m) 6 H, |
| ($CDCl_3$, TMS als | 2.23 - 2.57 (m) 4 H, |
| interner Standard) | 3.43 (s) 2 H, |
| | 3.92 (m) 2 H, |
| | 4.50 (m) 2 H, |
| | 5.93 (m) 2 H, |
| | 6.23 (breit) 3 H, austauschbar mit $D_2O$ |
| | 6.77 (s) 1 H, |
| | 6.80 - 7.33 (m) 4 H ppm. |

Beispiel 6

4-Amino-5-ethoxycarbonyl-3-[4-[3-(piperidinomethyl)phenoxy]but-2-enyl]amino-isothiazol-1,1-dioxid

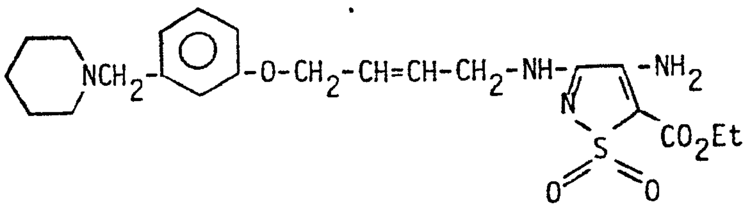

1.4 g (5.4 mmol) 1-Amino-4-[3-(piperidinomethyl)phenoxy]-2-buten und 1.3 g (5.4 mmol) 4-Amino-3-ethoxy-5-ethoxycarbonyl-isothiazol-1,1-dioxid in 35 ml Acetonitril werden 20 Std. bei Raumtemperatur gerührt. Der gebildete Niederschlag wird abgesaugt und im Vakuum getrocknet. 0.70 g (28 %) farblose Kristalle vom Schmelzpunkt 163 - 164 °C.

$C_{22}H_{30}N_4O_5S$ (462.6)

Rf ($CH_3OH/CH_2Cl_2$ 80 : 20): 0.4

| | |
|---|---|
| $^1$H-NMR-Daten: | $\delta$ = 1.26 (t) 3 H, |
| ($d_6$-DMSO, TMS als | 1.33 - 1.68 (m) 6 H, |
| interner Standard) | 2.22 - 2.50 (m) 4 H, |
| | 3.44 (s) 2 H, |
| | 4.03 - 4.23 (m) 2 H, |
| | 4.30 (q) 2 H, |
| | 4.52 - 4.73 (m) 2 H, |
| | 5.95 - 6.20 (m) 2 H, |
| | 6.82 - 7.52 (m) 4 H, |
| | 9.45 (breit) 3 H, austauschbar mit $D_2O$ ppm. |

Beispiel 7

N-[4-[3-(Piperidinomethyl)phenoxy]but-2-enyl]harnstoff

a) $N^1$-[4-[3-(Piperidinomethyl)phenoxy]but-2-enyl]-$N^2$-benzoyl-harnstoff
Zu einer Lösung von 1.31 g (5 mmol) 1-Amino-4-[3-(piperidinomethyl)phenoxy]-2-buten in 25 ml trockenem Toluol werden 0.73 g (5 mmol) Benzoylisocyanat in 10 ml trockenem Toluol langsam zugetropft. Nach 3 Std. bei Raumtemperatur wird die Lösung filtriert und im Vakuum eingedampft. Man erhält 2.03 g des Benzoylharnstoffs, die nach DC ($CH_2Cl_2$ / $CH_3OH$ 9 : 1) sauber sind. Schmelzpunkt 108 °C.

b) 1.77 g (4.35 mmol) des Benzoylharnstoffs und 0.55 g (4 mmol) Kaliumcarbonat werden in 100 ml Methanol und 10 ml Wasser 6 Std. bei 60 °C gerührt. Nach Einrotieren wird der Rückstand in 20 ml Wasser und 30 ml Methylenchlorid aufgenommen. Die Phasen werden getrennt, die wäßrige nochmals mit 30 ml Methylenchlorid extrahiert und die vereinigten organischen Phasen über Natriumsulfat getrocknet. Nach Eindampfen im Vakuum verbleibt ein farbloses Öl, das nach Anreiben mit Essigester kristallisiert (0.97 g). Nach Umkristallisieren aus wenig Essigester erhält man 0.44 g (34 %) farblose Kristalle, Schmelzpunkt 84 - 85 °C.

$C_{17}H_{25}N_3O_2$ (303.4)

Rf ($CH_2Cl_2$ / $CH_3OH$ / $N(C_2H_5)_3$ 80 : 20 : 1): 0.52

| | |
|---|---|
| $^1$H-NMR-Daten: | $\delta$ = 1.26 - 1.73 (m) 6 H, |
| ($CDCl_3$, TMS als | 2.21 - 2.51 (m) 4 H, |
| interner Standard) | 3.44 (s) 2 H, |
| | 3.68 - 3.92 (m) 2 H, |
| | 4.40 - 4.57 (m) 2 H, |
| | 5.01 (breit) 2 H, austauschbar mit $D_2O$ |
| | 5.73 (t) 1 H, austauschbar mit $D_2O$ |
| | 5.81 - 6.00 (m) 2 H, |
| | 6.70 - 7.37 (m) 4 H ppm. |

## Beispiel 8

1-Amino-1-[4-[3-(pyrrolidinomethyl)phenoxy]but-2-enyl]amino-2-nitro-ethylen

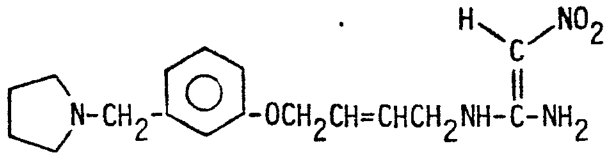

1.24 g (5.0 mmol) 1-Amino-4-[3-(1-pyrrolidinomethyl) phenoxy]-2-buten und 0.83 g (5.0 mmol) 1,1-S,S-Dimethyldithio-2-nitro-ethen werden in 25 ml Acetonitril 3 Std. unter Rückfluß gekocht. Der nach Abdampfen des Lösungsmittels im Vakuum verbleibende Rückstand wird in 50 ml Methanol aufgenommen und auf Rückflußtemperatur erhitzt. Während 2 Std. wird ein kräftiger Strom trockenen Ammoniaks eingeleitet. Nach Eindampfen der Lösung wird der Rückstand mit Methanol/Essigester 80:20 an Kieselgel gereinigt. Die Hauptfraktion ergibt nach Eindampfen 1.21 g (73 %) der Titelverbindung als blaßgelbes Öl.

$C_{17}H_{24}N_4O_3$ (332.4)

Rf ($EtOH/N(Et)_3$ 97 : 3 ): 0.50

| | |
|---|---|
| $^1$H-NMR-Daten: | $\delta$ = 1.60 - 1.91 (m) 4 H, |
| ($CDCl_3$, TMS als interner | 2.35 - 2.63 (m) 4 H, |
| Standard) | 3.57 (s) 2 H, |
| | 3.76 - 3.98 (m) 2 H, |
| | 4.38 - 4.57 (m) 2 H, |
| | 5.78 - 6.00 (m) 2 H, |
| | ~ 6.1 (breit) 3 H, (austauschbar mit $D_2O$) |
| | 6.70 (s) 1 H, |
| | 6.75 - 7.33 (m) 4 H ppm. |

0178503

## Beispiel 9

1-Methylamino-1-[4-[3-(pyrrolidinomethyl)phenoxy]but-2-enyl]amino-2-nitro-ethylen

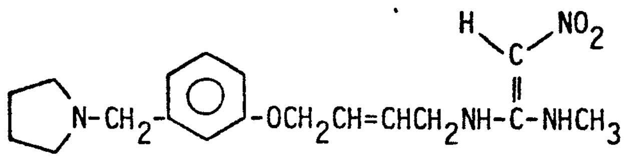

2.50 g (10.1 mmol) 1-Amino-4-[3-(1-pyrrolidinomethyl)phenoxy]-2-buten und 1.67 g (10.1 mmol) 1,1-S,S-Dimethyldithio-2-nitro-ethen werden in 30 ml Acetonitril 3 Std. gekocht. Nach Abdampfen des Lösungsmittels im Vakuum wird das verbleibende Öl in 60 ml Methanol aufgenommen. In die unter Rückfluß siedende Lösung wird während 2,5 Std. ein kräftiger Strom trockenen Methylamins eingeleitet. Nach Abdampfen des Lösungsmittels im Vakuum wird der Rückstand mit Methanol/Triethylamin 98 : 2 an Kieselgel chromatographiert. Nach Abdampfen des Lösungsmittels erhält man 2.76 g (79 %) der Titelverbindung als blaßgelbes Öl.

$C_{18}H_{26}N_4O_3$ (346.4)

Rf ($CH_3OH/N(Et)_3$ 98 : 2 ): 0.53

| | |
|---|---|
| $^1$H-NMR-Daten: | $\delta$ = 1.58 - 1.91 (m) 4 H, |
| ($CDCl_3$, TMS als interner | 2.35 - 2.67 (m) 4 H, |
| Standard) | 2.91 (s, breit) 3 H, |
| | 3.61 (s) 2 H, |
| | 3.80 - 4.10 (m) 2 H, |
| | 4.40 - 4.64 (m) 2 H, |
| | 5.81 - 6.07 (m) 2 H, |
| | 6.66 (s) 1 H, |
| | 6.72 - 7.42 (m) 4 H, |
| | 10.27 (breit) 2 H (austauschbar mit $D_2O$) ppm. |

## PATENTANSPRÜCHE

1. Butenylderivate der allgemeinen Formel I

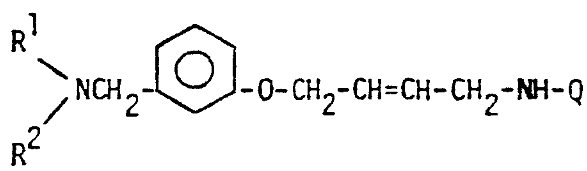

(I)

in der $R^1$ und $R^2$, die gleich oder verschieden sein können, jeweils für Wasserstoff, lineares oder verzweigtes $C_1$-$C_6$-Alkyl oder $C_5$-$C_6$-Cycloalkyl stehen oder $R^1$ und $R^2$ zusammen mit dem Stickstoffatom, an das sie gebunden sind, einen 4-bis 7gliedrigen stickstoffhaltigen alicyclischen Heterocyclus bilden, Q für die Gruppierung

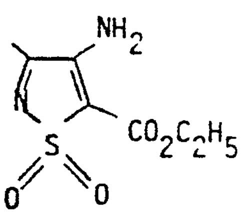

oder

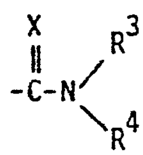

steht, worin X ein Sauerstoffatom oder die Gruppe N-CN oder $CH$-$NO_2$ be-

deutet, $R^3$ und $R^4$ unabhängig voneinander jeweils für ein Wasserstoffatom, eine unsubstituierte oder durch eine Hydroxylgruppe substituierte lineare oder verzweigte $C_1$-$C_6$-Alkylgruppe oder eine Propargylgruppe stehen oder $R^3$ und $R^4$ zusammen mit dem Stickstoffatom, an das sie gebunden sind, einen 4- bis 6gliedrigen alicyclischen stickstoffhaltigen Heterocyclus bilden, sowie ihre physiologisch annehmbaren Hydrate und Salze.

2. Butenylverbindungen nach Anspruch 1, dadurch gekennzeichnet, daß $R^1$ für Wasserstoff oder $C_1$-$C_3$-Alkyl steht und $R^2$ für $C_5$-$C_6$-Cycloalkyl oder $C_1$-$C_3$-Alkyl steht oder $R^1$ und $R^2$ zusammen mit dem Stickstoffatom, an das sie gebunden sind, einen unsubstituierten 4- bis 7gliedrigen alicyclischen Heterocyclus bilden.

3. Butenylverbindungen nach Anspruch 1 oder 2, dadurch gekennzeichnet, daß $R^3$ ein Wasserstoffatom und $R^4$ eine Methylgruppe bedeutet.

4. 1-Methylamino-1-[4-[3-(piperidinomethyl)phenoxy]but-2-enyl]amino-2-nitro-ethylen und die physiologisch annehmbaren Salze davon.

5. $N^1$-Cyano-$N^2$-methyl-$N^3$-[4-[3-(piperidinomethyl)phenoxy]but-2-enyl]guanidin und die physiologisch annehmbaren Salze davon.

6. Verfahren zur Herstellung von Butenylverbindungen nach den Ansprüchen 1 bis 5, dadurch gekennzeichnet, daß man

a) zur Herstellung von Verbindungen, bei denen $R^1$ und $R^2$ wie in Anspruch 1 definiert sind und Q für die Gruppierung

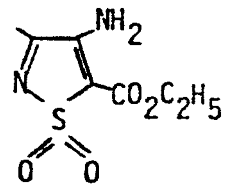

steht, ein Amin der allgemeinen Formel II

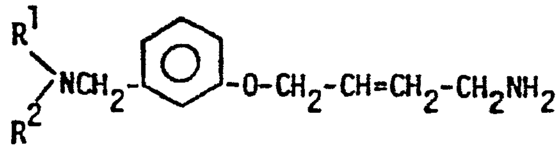 (II)

in der $R^1$ und $R^2$ wie in Anspruch 1 definiert sind, mit einem Isothiazolderivat der Formel

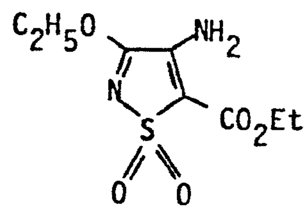

zu einer erfindungsgemäßen Verbindung der allgemeinen Formel I umsetzt, oder daß man

b) zur Herstellung von Verbindungen, bei denen $R^1$ und $R^2$ wie in Anspruch 1 definiert sind und Q für die Gruppierung

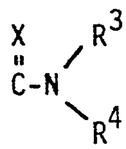

steht, worin X für N-CN oder $CH-NO_2$ steht, eine Verbindung der allgemeinen Formel III

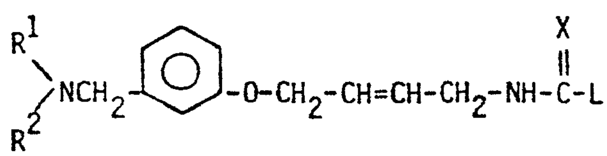 (III)

in der $R^1$ und $R^2$ wie in Anspruch 1 definiert sind und L für $SCH_3$, $OCH_3$, $OC_2H_5$ oder O-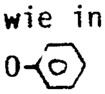 als Austrittsgruppe steht, mit einem Amin der allgemeinen Formel IV

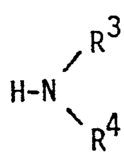 (IV)

in der $R^3$ und $R^4$ die in Anspruch 1 angegebenen Bedeutungen haben, zu einer erfindungsgemäßen Verbindung der allgemeinen Formel I umsetzt, oder daß man

c) zur Herstellung von Verbindungen, bei denen $R^1$ und $R^2$ wie in Anspruch 1 definiert sind und Q für die Gruppierung

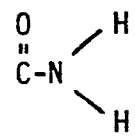

steht, eine Verbindung der allgemeinen Formel V

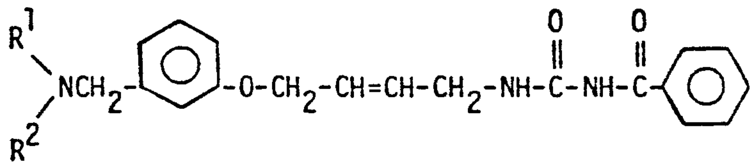 (V)

in der $R^1$ und $R^2$ die in Anspruch 1 angegebenen Bedeutungen haben, zu einer erfindungsgemäßen Verbindung der allgemeinen Formel I umsetzt,

und daß man gegebenenfalls die gemäß a) bis c) erhaltene Verbindung in an sich bekannter Weise in ihr physiologisch annehmbares Salz umwandelt.

7. Arzneimittel, dadurch g e k e n n z e i c h n e t , daß es eine Verbindung nach einem der Ansprüche 1 bis 5 zusammen mit einem inerten, pharmazeutisch annehmbaren Träger oder Verdünnungsmittel enthält.

# PATENTANSPRÜCHE

## für den Vertragsstaat AT

1. Verfahren zur Herstellung von Butenylderivaten der allgemeinen Formel I

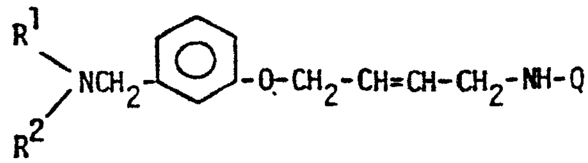

(I)

in der $R^1$ und $R^2$, die gleich oder verschieden sein können, jeweils für Wasserstoff, lineares oder verzweigtes $C_1$-$C_6$-Alkyl oder $C_5$-$C_6$-Cycloalkyl stehen oder $R^1$ und $R^2$ zusammen mit dem Stickstoffatom, an das sie gebunden sind, einen 4- bis 7gliedrigen stickstoffhaltigen alicyclischen Heterocyclus bilden, Q für die Gruppierung

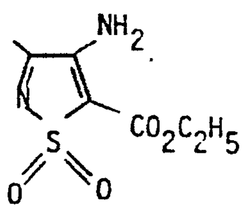

oder

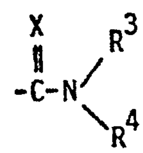

steht, worin X ein Sauerstoffatom oder die Gruppe N-CN oder $CH-NO_2$ bedeutet, $R^3$ und $R^4$ unabhängig voneinander jeweils für ein Wasserstoffatom, eine unsubstituierte oder durch eine Hydroxylgruppe substituierte lineare oder verzweigte $C_1$-$C_6$-Alkylgruppe oder eine Propargylgruppe stehen oder $R^3$ und $R^4$ zusammen mit dem Stickstoffatom, an das sie gebunden sind, einen 4- bis 6gliedrigen alicyclischen stickstoffhaltigen Heterocyclus bilden, sowie ihrer physiologisch annehmbaren Hydrate und Salze, dadurch g e k e n n z e i c h n e t , daß man

a) zur Herstellung von Verbindungen, bei denen $R^1$ und $R^2$ wie oben definiert sind und Q für die Gruppierung

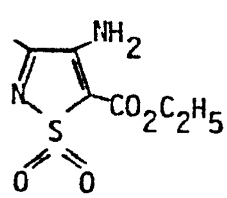

0178503

steht, ein Amin der allgemeinen Formel II

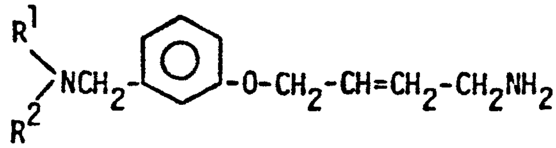 (II)

in der $R^1$ und $R^2$ wie oben definiert sind, mit einem Isothiazolderivat der Formel

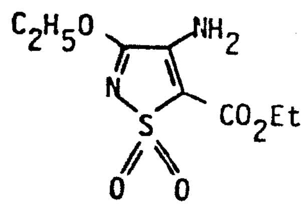

zu einer erfindungsgemäßen Verbindung der allgemeinen Formel I umsetzt, oder daß man

b) zur Herstellung von Verbindungen, bei denen $R^1$ und $R^2$ wie oben definiert sind und Q für die Gruppierung

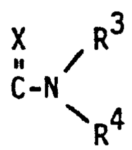

steht, worin X für N-CN oder $CH-NO_2$ steht, eine Verbindung der allgemeinen Formel III

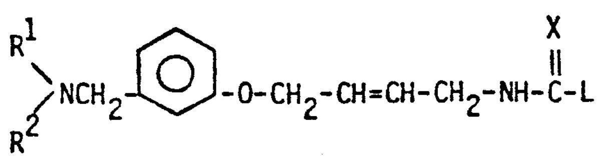 (III)

in der $R^1$ und $R^2$ wie oben definiert sind, und L für $SCH_3$, $OCH_3$, $OC_2H_5$ oder  als Austrittsgruppe steht, mit einem Amin der allgemeinen Formel IV

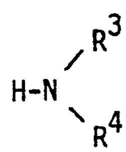 (IV)

in der $R^3$ und $R^4$ die oben angegebenen Bedeutungen haben, zu einer erfindungsgemäßen Verbindung der allgemeinen Formel I umsetzt, oder daß man

c) zur Herstellung von Verbindungen, bei denen $R^1$ und $R^2$ wie oben definiert sind und Q für die Gruppierung

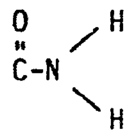

steht, eine Verbindung der allgemeinen Formel V

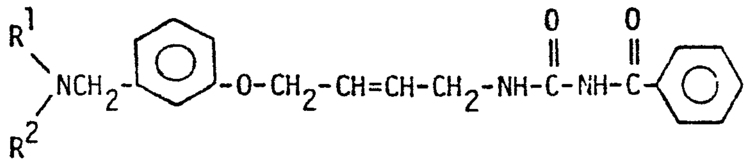

(V)

in der $R^1$ und $R^2$ die oben angegebenen Bedeutungen haben, zu einer erfindungsgemäßen Verbindung der allgemeinen Formel I umsetzt,

und daß man gegebenenfalls die gemäß a) bis c) erhaltene Verbindung in an sich bekannter Weise in ihr physiologisch annehmbares Salz umwandelt.

2. Verfahren nach Anspruch 1 zur Herstellung von Verbindungen, bei denen $R^1$ für Wasserstoff oder $C_1$-$C_3$-Alkyl steht und $R^2$ für $C_5$-$C_6$-Cycloalkyl oder $C_1$-$C_3$-Alkyl steht oder $R^1$ und $R^2$ zusammen mit dem Stickstoffatom, an das sie gebunden sind, einen unsubstituierten 4- bis 7gliedrigen alicyclischen Heterocyclus bilden.

3. Verfahren nach Anspruch 1 oder 2 zur Herstellung von Verbindungen, bei denen $R^3$ ein Wasserstoffatom und $R^4$ eine Methylgruppe bedeutet.

4. Verfahren nach Anspruch 1 zur Herstellung von 1-Methylamino-1-[4-[3-(piperidinomethyl)phenoxy]but-2-enyl]amino-2-nitro-ethylen und der physiologisch annehmbaren Salze davon.

5. Verfahren nach Anspruch 1 zur Herstellung von $N^1$-Cyano-$N^2$-methyl-$N^3$-[4-[3-(piperidinomethyl)phenoxy]but-2-enyl]guanidin und der physiologisch annehmbaren Salze davon.

Europäisches Patentamt

# EUROPÄISCHER RECHERCHENBERICHT

0178503

Nummer der Anmeldung

EP 85 11 2140

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
| Y | CHEMICAL ABSTRACTS, Band 98, Nr. 7, 14. Februar 1983, page 627, Nr. 53436b, Columbus, Ohio, US; & JP - A - 57 165 348 (TEIKOKU HORMONE MFG. CO., LTD.) 12-10-1982 | 1,6,7 | C 07 D 295/00<br>C 07 D 275/02<br>A 61 K 31/395<br>A 61 K 31/425 |
| Y | EP-A-0 067 436 (MERCK) * Ansprüche; Seite 17 * | 1,6,7 | |
| Y | EP-A-0 029 306 (GLAXO) * Ansprüche * | 1,6,7 | |
| | | | RECHERCHIERTE SACHGEBIETE (Int. Cl 4) |
| | | | C 07 D 295/00<br>C 07 D 275/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 24-01-1986 | MOREAU J.M. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument